# EUROPEAN PATENT APPLICATION

(11) **EP 1 430 836 A1**
(43) Date of publication of application: **23.06.2004**
(21) Application number: 03078907.7
(22) Date of filing: 08.12.2003
(51) Int. Cl.: A61B 6/14, A61B 6/00

(54) **Incorporation of a density target with dental films to facilitate subtractive radiography**

(30) Priority: 20.12.2002 US 324801
(71) Applicant: EASTMAN KODAK COMPANY, Rochester, New York 14650 (US)
(72) Inventor: Squilla, John R., c/o Eastman Kodak Company, Rochester New York 14650-2201 (US); Boland, John T., c/o Eastman Kodak Company, Rochester New York 14650-2201 (US); Spoonhower, John P., c/o Eastman Kodak Company, Rochester New York 14650-2201 (US)
(74) Representative: Haile, Helen Cynthia

(57) **Abstract**

A method and system for equalizing non-diagnostic differences that occur in two or more radiographic images taken of the same object at different times utilizes a radiation source that generates a beam of radiation and an image receiver positioned to receive radiation from the radiation source that interacts with the object, whereby image data of the object is captured by the receiver. The method includes the steps of interposing a target in the path of the beam of radiation between the source and the image receiver such that the target is imaged upon the image receiver together with the object; using the radiation source and the receiver to capture two or more images of the object at different times; generating measurements of the targets in each of the captured images; and using the measurements to equalize the image data of the radiographic images, thereby generating two or more equalized images that have been processed to equalize the non-diagnostic differences between the images.

## Description

The invention relates generally to the field of dental radiography, and in particular to the field of subtractive radiography.

Radiography is a familiar and well-developed process used in support of medical practice, and has proven to be very useful in applications such as mammography and dentistry, specifically for the detection of abnormal tissue or infections, as well as for monitoring changes over time. A specific monitoring application is subtractive radiography, wherein two or more radiographic images are compared using computer software to detect changes over time. An important inhibiting factor in subtractive radiography is the possibility of non-diagnostic density differences between the radiographic images due to a number of sources, including variations in the film, illumination differences, incidence angle of the x-ray source, and exposure and development differences. Any such differences must be corrected in order for the subtractive radiography process to reveal true changes over time.

In U.S. Patent 5,544,238, Galkin describes a method for correcting the effect of image quality of a film processor that develops a radiographic image. Galkin describes a mammography application that uses a film cassette with an intensifying screen to form the image. After the radiographic image is captured, the film is removed from its cassette in a darkroom so that a graded density pattern can be impressed on a reserved and protected area of the film, in this case using a visible light exposure. The density pattern includes a plurality of symbols that are compared to a corresponding set on a control film allowing the uncorrected film to be corrected to an ideal condition, and ultimately to characterize film processor performance.

While in Galkin the film ends up with a graded density pattern on it, the pattern is not exposed at the time of radiographic image capture and thus does not include the non-diagnostic density influences imparted during the radiographic capture process. What is needed, therefore, is a method and system for recording and measuring the non-diagnostic density differences at the time of the radiographic image capture, and especially as between a series of two or more such image captures.

The present invention is directed to overcoming one or more of the problems set forth above. Briefly summarized, according to one aspect of the present invention, a method and system for equalizing non-diagnostic differences that occur in two or more radiographic images taken of the same object at different times utilizes a radiation source that generates a beam of radiation and an image receiver positioned to receive radiation from the radiation source that interacts with the object, whereby image data of the object is captured by the receiver. The method includes the steps of interposing a target in the path of the beam of radiation between the source and the image receiver such that the target is imaged upon the image receiver together with the object; using the radiation source and the receiver to capture two or more images of the object at different times; generating measurements of the targets in each of the captured images; and using the measurements to equalize the image data of the radiographic images, thereby generating two or more equalized images that have been processed to equalize the non-diagnostic differences between the images.

From another aspect, the invention comprises a target for use in subtractive radiography for equalizing non-diagnostic differences that occur in two or more radiographic images taken of the same object at different times, wherein the radiographic images are obtained by exposing an image receiver to a beam of radiation that interacts with the object. According to this aspect, the target comprises a variable density element supported directly upon the image receiver such that the variable density element is imaged upon the image receiver simultaneously with the exposure of the object. In yet another aspect, the target comprises a variable density element supported in a spaced relationship with respect to the image receiver such that the variable density element is imaged upon the image receiver simultaneously with the exposure of the object.

The advantage of the invention lies in correction of non-diagnostic density differences between the radiographic images due to sources such as variations in the film, illumination differences, incidence angle of the x-ray source, and exposure and development differences. Such corrected images can then be employed advantageously in a subtractive radiography process.

These and other aspects, objects, features and advantages of the present invention will be more clearly understood and appreciated from a review of the following detailed description of the preferred embodiments and appended claims, and by reference to the accompanying drawings.
FIG. 1 is a pictorial view of a radiography system incorporating a density target in accordance with the invention to facilitate subtractive radiography.
FIG. 2 shows a bitewing intraoral film positioner with an aiming ring as known in the prior art.
FIG. 3 shows the bitewing positioner of Figure 2 incorporating the density target as shown in Figure 1 as an additional surface.
FIG. 4 shows the bitewing positioner of Figure 2 incorporating the density target as shown in Figure 1 as a target surface applied directly to the radiographic film.
FIG. 5 shows a bitewing positioner incorporating a density target as shown in Figure 3 but without an aiming ring.
FIG. 6 shows a bitewing positioner incorporating a density target as shown in Figure 4 but without an aiming ring.
FIG. 7 is a flowchart of the process for utilizing the density targets to equalize images taken at different times.
FIG. 8 is a flowchart of the process shown in Figure 7, further illustrating network connectivity of a portion of the process.

Because dental radiography and systems employing subtractive radiography are well known, the present description will be directed in particular to elements forming part of, or cooperating more directly with, system and method in accordance with the present invention. Elements not specifically shown or described herein may be selected from those known in the art. Certain aspects of the embodiments to be described may be provided in software. Given the system and method as shown and described according to the invention in the following materials, software not specifically shown, described or suggested herein that is useful for implementation of the invention is conventional and within the ordinary skill in such arts.

Still further, as used herein, the computer program may be stored in a computer readable storage medium, which may comprise, for example; magnetic storage media such as a magnetic disk (such as a hard drive or a floppy disk) or magnetic tape; optical storage media such as an optical disc, optical tape, or machine readable bar code; solid state electronic storage devices such as random access memory (RAM), or read only memory (ROM); or any other physical device or medium employed to store a computer program.

The subject invention can be used with either indirect or direct radiography systems (i.e., either the use or non-use of an intensifying screen, respectively). For the purpose of this disclosure, indirect radiography includes image receivers such as an x-ray film or a scanned storage phosphor screen of the type used in computed radiography; in each case, the receiver has to be processed to obtain a readable image. Direct radiography includes direct capture technology, where a readable digital radiographic image is directly captured by a digital radiographic sensor, e.g., an electronic matrix, such as an amorphous selenium detector array. Although in the preferred embodiment the image receiver is described in terms of an x-ray film, this is not intended as a limitation and the claims are intended to cover the use of any kind of direct or indirect receiver. The purpose of the method is to correct film-based or digital radiographic images of the same patient, taken at different times, allowing a subtractive radiography process, or visual inspection, to reveal/detect true changes in the patient's tissue.

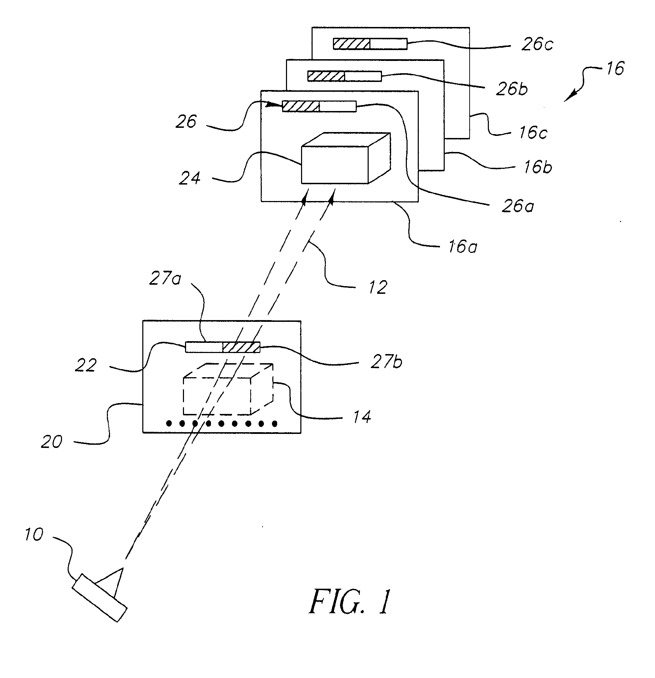

The general concept of the invention is shown in Figure 1, where a radiation source 10 generates an x-ray beam 12 that images a bodily object or area, such a tooth 14, upon an x-ray film 16. For subtractive radiography, several images of the same image will be taken over time in an attempt to reveal changes in the image over time. As heretofore mentioned, an important inhibiting factor in subtractive radiography is the possibility of non-diagnostic density differences between the radiographic images due to a number of sources, including variations in the film, illumination differences, incidence angle of the x-ray source, and exposure and development differences.

To deal with these differences, and in accordance with the invention, a target 20 is interposed in the path of the x-ray beam 14 such that a graded density pattern, such as a density step wedge 22, formed on the target is imaged on the x-ray film 16 together with the desired object. More specifically, an image 24 of the tooth and an image 26 of the step wedge is formed on the x-ray film 16. When this is done for a plurality of film images 16a, 16b, 16c... over time, the density changes in the step wedge between the step wedge images 26a, 26b, 26c... will reveal the non-diagnostic density differences for the series of images taken over time of the same object. In the preferred embodiment, the x-ray density step wedge 26 comprises two or more density levels and is exposed simultaneously with the patient tissue (as shown in Figure 1).

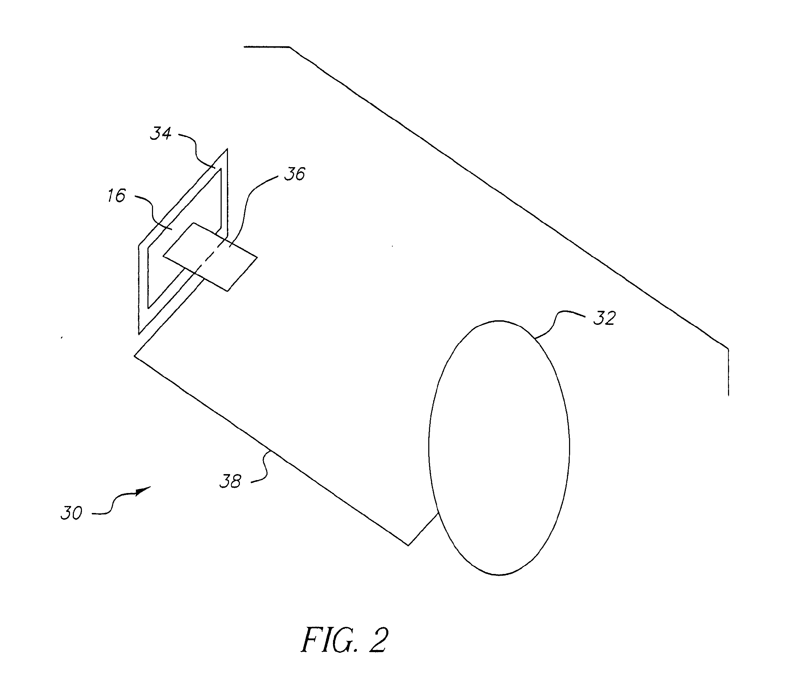

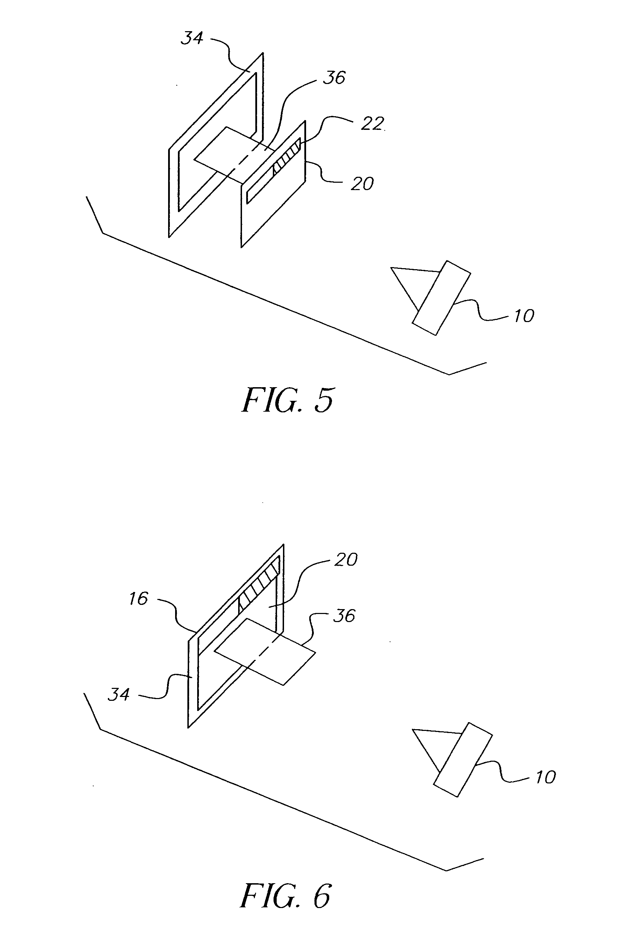

An x-ray film is typically positioned in the mouth of a patient by means of a conventional intra-oral bitewing film positioner 30, such as shown with an x-ray aiming ring 32 in Figure 2. The positioner 30 includes a bitewing package 34 enclosing and protecting the x-ray film 16 and a bite plate 36, which is gripped between the clenched teeth of the patient. The x-ray aiming ring 32 is separated from the bitewing package 34 by an extension 38 that places the aiming ring 32 outside the patient's mouth, which facilitates orientation of the x-ray source 10 toward an intra-oral object that is temporarily obscured by the clenched jaws of the patient. In many situations, as will be shown in Figures 5 an 6, the x-ray aiming ring 32 and its associated extension 38 may be omitted.

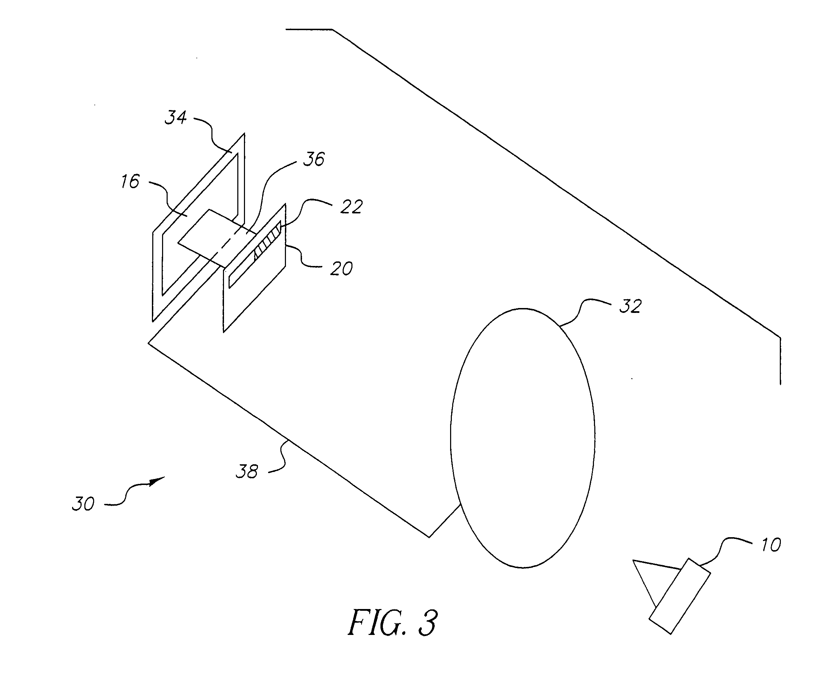

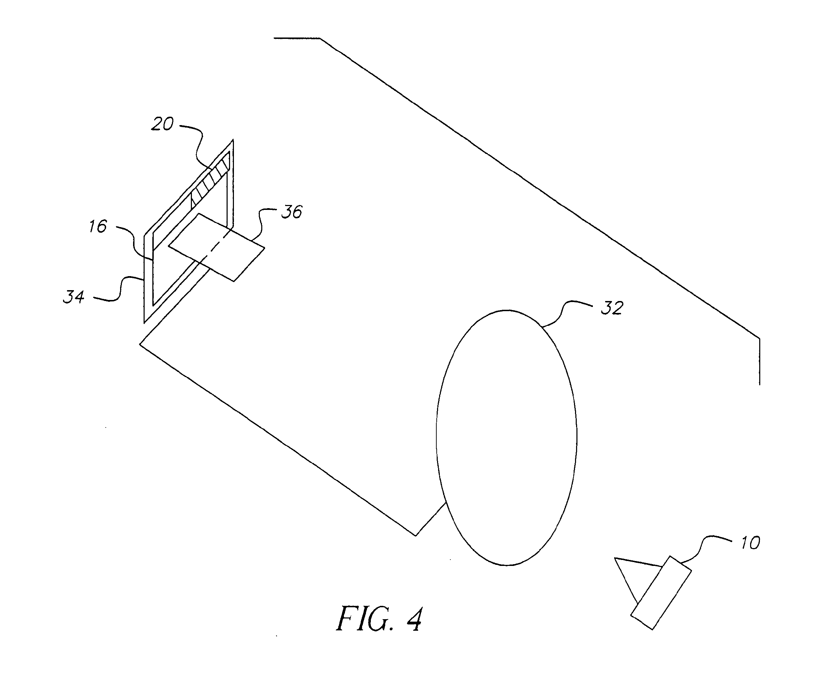

As shown in Figures 4 and 6, the x-ray density step wedge 22 is applied directly to the radiographic film 16. As shown in Figures 3 and 5, the x-ray density wedge 22 is applied to an additional target surface 30 that is supported apart from the bitewing package 34 by, e.g., attachment to the bitewing plate 36. In both cases the wedge 22 is situated to the side of the material exposed to the x-ray source. The step wedge 22 is positioned so as to affect a portion of the radiographic image that does not overlap the image of the tissue being examined. Two density levels 27a and 27b are used to provide repeatable minimum and maximum exposures that are used by computer software to adjust the radiographic images to each other (typically one image is adjusted to the other, although each could be adjusted to standard values) through linear, dynamic range adjustment. Additional levels are used to provide more detailed adjustment through piecewise linear adjustment or curve-fitting methods.

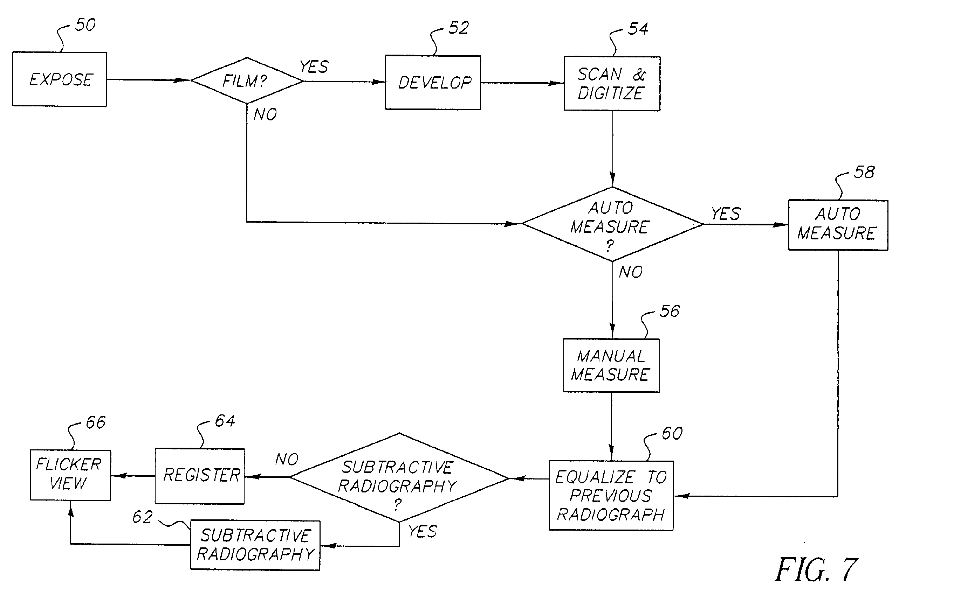

Figure 7 is a flowchart of the process for utilizing the density information of the step wedges. The tissue and target are exposed simultaneously in an exposure step 50. If a photographic film is used, then the film is developed in a development stage 52 and scan/digitized in an analog to digital conversion 54 to bring it into digital format. Once in digital format, the image 26 of the step wedge 22 is measured either manually by an operator measurement 56 or automatically by a computer software based stage 58. The computer software based measurement is facilitated by determining the coordinates of the step wedge, from which the signal values of the densities within the area defined by the coordinates are determined. The measurements of the step wedge are then used in a processing stage 60 to equalize the radiograph to a previous image that has been through the same steps. At this point the user may choose to use a subtractive radiography process 62, wherein computer software is used to register, compare, and measure changes between the two radiographs, or a registration process 64 may be chosen, followed by visual inspection in a viewing stage 66.

Registration of images taken at different times in the registration process 64 can be accomplished interactively using a process similar to that described in accordance with the cross-referenced commonly assigned copending application Serial No. 09/970,243, which is incorporated herein by reference, and in which a series of comparative views of related images are produced and presented to a user through a graphical user interface presented at the viewing stage 66. More specifically, these comparative views enable user-friendly registration of the images prior to engaging in a subtractive process for isolating changes between the images. Automatic registration can be accomplished via software by detecting and recognizing unique points on the object as they appear in each x-ray, using either existing points or points purposefully added to the object for this purpose. Alternatively, automatic registration can be accomplished via software by matching the shape, or outline, of the dental object in each x-ray.

This invention is intended to enable removal of non-diagnostic irregularities prior to a subtractive radiography process. In a subtractive process of this type, subtracting one image from another effectively removes from the difference image all features that do not change, while highlighting or otherwise denoting those that do. Details of such a subtractive process, though not used in connection with radiography, are disclosed in U. S. Patent No. 6,163,620, which is incorporated herein by reference. In a dental environment, this process can be used to isolate various types of temporal changes between radiographs of the same object taken at different times, e.g., to isolate bone loss due to periodontal disease (by looking under the gum line). It should be understood, however, that the viewing stage 66 is capable of producing a visual "differencing" effect (i.e., flickering) between the two images that may be sufficient in some cases to indicate the temporal change between the two images.

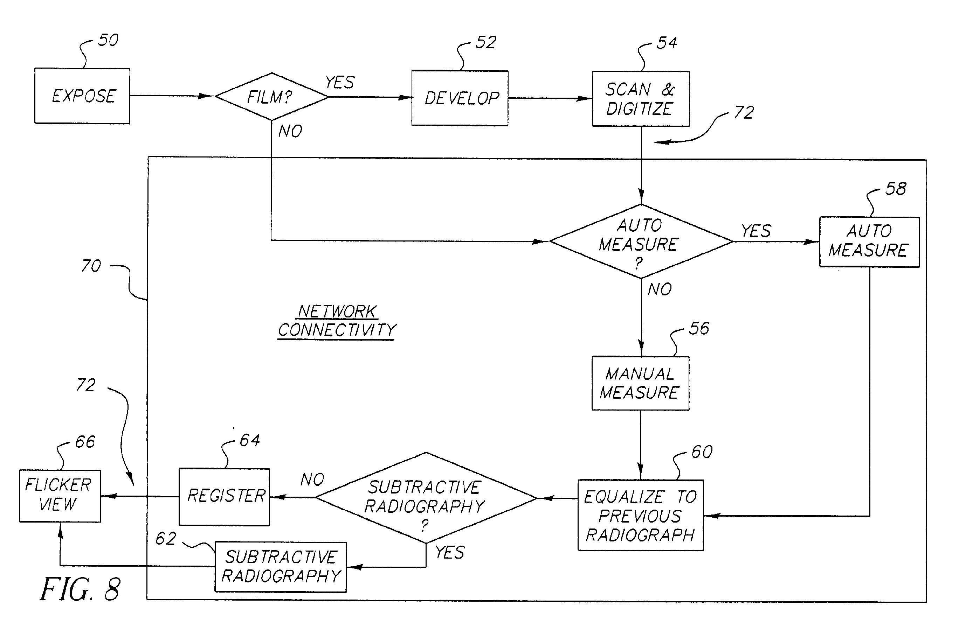

In a typical implementation of the invention, the computer program product bearing the inventive algorithms would either be available directly to a user, who would use it in connection with the user's processing of images, or it would be used in a centralized setting, where a user would bring radiographs to a work station for scanning and digitization, or would directly enter digital scan data into the work station. Alternatively, the algorithms could be made available in a web-based version of the product, where either the algorithms are downloaded via a network connection to the user or the algorithm computation is done on a server in a web-based environment. In the latter case, Figure 8 shows that once the images are in digital form, the measurement, equalization, and registration / subtractive radiography steps identified in a network connectivity stage 70 may be performed across a network connection 72 to a server or remote provider, wherein, e.g., the analog to digital conversion 54 and the viewing stage 66 would be undertaken in a browser-enabled client setting and the network connectivity portion would be undertaken in a server setting.

The invention has been described with reference to a preferred embodiment. However, it will be appreciated that variations and modifications can be effected by a person of ordinary skill in the art without departing from the scope of the invention. For instance, it would be apparent to the skilled person that other types of radiation, such as ultrasonic radiation, could be used to advantage according to the invention. Furthermore, the non-diagnostic differences could be electrical noise-based differences rather than differences associated with image densities. The invention should also be understood to apply without limitation to any type of radiographic exploration of the human body, or any other kind of object that is subject to change over time, and not just to a dental application.

## Claims

1. A method for equalizing non-diagnostic differences that occur in two or more radiographic images taken of the same object at different times, said method comprising the steps of:
providing and positioning a radiation source that generates a beam of radiation;
positioning an image receiver to receive radiation from the radiation source that interacts with the object, whereby image data of the object is captured by the receiver;
interposing a target in the path of the beam of radiation between the source and the image receiver such that the target is imaged upon the image receiver together with the object;
using the radiation source and the receiver to capture two or more images of the object at different times;
generating measurements of the targets in each of the captured images; and
using the measurements to equalize the image data of the radiographic images, thereby generating two or more equalized images that have been processed to equalize the non-diagnostic differences between the images.

2. The method as claimed in claim 1 wherein the equalized images are used in a subtractive radiography process.

3. The method as claimed in claim 1 wherein the target is a graded density step wedge having at least two different densities.

4. The method as claimed in claim 1 wherein the target is positioned between the radiation source and the object.

5. The method as claimed in claim 1 wherein the target is positioned between the object and the image receiver.

6. The method as claimed in claim 1 wherein the target is positioned on the image receiver.

7. The method as claimed in claim 1 wherein the target is a graded density pattern having at least two different densities; the image receiver is a radiographic film; the source is an x-ray source; and the non-diagnostic differences are density differences due to variations in the film, illumination differences of the x-ray source, incidence angle of the x-ray source, or exposure and development differences related to development of the film.

8. The method as claimed in claim 1 wherein the image receiver is an x-ray film.

9. The method as claimed in claim 1 wherein the image receiver is an indirect radiographic sensor.

10. The method as claimed in claim 1 wherein the image receiver is a direct radiographic sensor.
